# EUROPÄISCHE PATENTSCHRIFT

(11) **EP 2 999 402 B1**
(45) Veröffentlichungstag und Bekanntmachung des Hinweises auf die Patenterteilung: **04.01.2023**
(21) Anmeldenummer: 14725192.0
(22) Anmeldetag: 21.05.2014
(51) Int. Cl.: A61B 5/00, A61B 5/03, A61B 5/1455, G01N 21/31

(54) **MESSSYSTEM UND -VERFAHREN ZUR MESSUNG VON PARAMETERN IN EINEM KÖRPERGEWEBE**
MEASUREMENT SYSTEM AND METHOD FOR MEASURING PARAMETERS IN A BODY TISSUE
SYSTÈME ET PROCÉDÉ DE MESURE DE PARAMÈTRES DANS UN TISSU CORPOREL

(30) Priorität: 22.05.2013 CH 9972013
(43) Veröffentlichungstag der Anmeldung: 30.03.2016
(73) Patentinhaber: Luciole Medical AG, 8048 Zürich (CH)
(72) Erfinder: MUSER, Markus Hugo, CH-8820 Wädenswil (CH); FRÖHLICH, Jürg Hans, CH-8006 Zürich (CH); BAUMANN, Dirk, CH-8008 Zürich (CH)
(74) Vertreter: BOVARD AG
(86) Internationale Anmeldenummer: PCT/EP2014/060414
(87) Internationale Veröffentlichungsnummer: WO 2014/187849

(56) Entgegenhaltungen:
- WO-A1-01/40776
- JP-A- 2002 010 986
- US-A- 5 706 821
- US-A1- 2004 054 290
- US-A1- 2005 043 596
- US-A1- 2007 167 867
- US-A1- 2008 077 023
- US-A1- 2013 072 807
- US-B1- 6 195 574
- GEORGE THEMELIS ET AL: "Near-infrared spectroscopy measurement of the pulsatile component of cerebral blood flow and volume from arterial oscillations", JOURNAL OF BIOMEDICAL OPTICS, Bd. 12, Nr. 1, 1. Januar 2007 (2007-01-01) , Seite 014033, XP055094642, ISSN: 1083-3668, DOI: 10.1117/1.2710250

## Beschreibung

Die vorliegende Erfindung betrifft ein Messsystem und ein Verfahren zur Bestimmung von Parametern eines Körpergewebes, wie etwa die Bestimmung von Blutvolumen und Blutfluss im Hirn oder der Muskulatur oder die Bestimmung eines Druckparameters. Insbesondere betrifft die Erfindung eine nicht-invasive Messung von zerebralen und muskulären Parametern.

Der Inhalt des Hirnschädels setzt sich aus den nicht-kompressiblen Komponenten Gehirn, zerebrales Blutvolumen (cerebral blood volume; CBV) und Liquor cerebrospinalis (Liquorraum) zusammen. Raumfordernde Prozesse führen nach Ausschöpfung der intrakraniellen Kompensationsmechanismen, wie z. B. Reduktion der Liquorproduktion, Verminderung des CBV, zu einer intrakraniellen Drucksteigerung. Dieser Sachverhalt geht z. B. aus der Monroe-Kellie Doktrin hervor. Bei Ansteigen des intrakraniellen Drucks (intracranial pressure; ICP) über 50 mmHg kann der zerebrale Perfusionsdruck (cerebral perfusion pressure; CPP) nicht mehr aufrechterhalten werden und es kommt durch eine globale zerebrale Ischämie zur zusätzlichen Schädigung von Hirnarealen, wie z. B. aus "Analysis of Intracranial Pressure: Past, Present and Future", A. Di leva et al; The Neuroscientist; 6. Februar 2013, bekannt ist. Dabei wird der CPP als Differenz des mittleren arteriellen Blutdruckes und des ICP ermittelt. Unter dem intrakraniellen Druck, ICP, versteht man definitionsgemäss den supratentoriellen Liquordruck, d. h. den Druck in den Seitenventrikeln und im Subarachnoidalraum über der Grosshirnkonvexität. ICP-Anstiege beruhen nach herkömmlicher Ansicht entweder auf einer Hirnschwellung, z. B. bei einer defekten Blut-Hirn-Schranke, oder einem zytotoxischen Hirnödem, wie etwa einer intrazellulären Wasserakkumulation, einer zusätzlichen Raumforderung, z. B. durch Tumor, Blutung, etc., oder einer Liquor-, Zirkulations- bzw. Resorptionsstörung.

Aus dem Stand der Technik sind verschiedene Methoden bekannt, den intrakraniellen Druck im Schädel und indirekt daraus ableitbare Parameter zu messen. Traditionell werden hierzu invasive Verfahren verwendet, bei welchen eine Messsonde durch den Schädel in das Hirngewebe eingeführt wird. Invasive Verfahren haben jedoch verschiedene Nachteil, wie unerwünschte Blutungen, aufwendige Durchführung der Verfahren, lokal beschränkte Messbereiche, etc.

Ferner werden nicht-invasive Messverfahren verwendet, die z. B. auf Ultraschall und Doppler-Effekt Methoden basieren und eine Korrelation zwischen einem erhöhten intrakraniellen Druck und der Pulsatilität nutzen. Es besteht jedoch auch bei gesunden Menschen eine starke Varianz beim Pulsatilitätsindex. Bei einem anderen Verfahren werden Prinzipien der Fluidmechanik und der Neurophysiologie mit Ergebnissen aus Magnetresonanzaufnahmen kombiniert, wie etwa beschrieben bei "Noninvasive intracranial compliance and pressure based on dynamic magnetic resonance imaging of blood flow and cerebrospinal flow; Patricia B. Raksin et al.; Neurosurg. Focus, Volume 14". Aus der EP 0933061 B1 ist eine Messanordnung bekannt, bei der eine Proportionalität zwischen dem Schädelinnendruck und einem im Gehörgang bestehenden Druck zur Bestimmung des intrakraniellen Drucks genutzt wird. Ferner wird in der US 2012/0136240 A1 ein System gezeigt, bei dem eine kontrollierte Verengung der Jugularvene zu einer Veränderung des Blutausflusses führt und der Druck des Blutausflusses gemessen wird, wobei aus der Relation zwischen Verengung und Blutfluss der intrakranielle Druck ermittelt wird. Dabei werden z. B. nahinfrarot (NIR) Sensoren zur Ermittlung des Blutausflusses verwendet. Ferner sind Absorptionsmessverfahren im Nahinfrarotbereich bekannt, um Konstituenten eines Organs, z. B. des Gehirns, zu überwachen, insbesondere zur Überwachung von oxygeniertem und desoxygeniertem Blut, oder eine Konzentration von oxygeniertem und desoxygeniertem Blut z. B. im Gehirn zu bestimmen, wie in US 6195574 oder US 5706821 gezeigt ist. Die JP 2002-010986 A beschreibt die gleichzeitige Messung des Intrakranialdrucks und eines Plethysmogramms. Aus den beiden Messkurven kann auf elastische Eigenschaften der Blutgefässe im Gehirn geschlossen werden. Die US 2008/077023 A1 beschreibt ein optisches Messverfahren, aus dem unter Zuhilfenahme zusätzlicher Messungen oder der Bestimmung von Wellenlängenvariationen auf einen Schädelinnendruck geschlossen wird.

Eine vergleichbare Situation ist bei Verletzungen und Traumen von Muskeln zu finden, die streng in Faszien angeordnet sind, wie z. B. ein Unterschenkel- oder Unterarmmuskel. Nach einem Trauma besteht durch einen kompartimental erhöhten Druck innerhalb einer Fascie ein hohes Risiko, dass der Muskel abstirbt, da eine Durchblutung des Muskels durch den Druckanstieg vermindert wird. Die Patienten erleiden einen Verlust der Muskulatur oder gar ein lebensbedrohliches Rhabdomyolyse-Syndrom. Es ist daher wünschenswert die Druckparameter in der Muskulatur zu bestimmen und überwachen zu können.

Bei den bekannten Verfahren sind zur Ermittlung von Druckparametern im Körpergewebe aufwendige Algorithmen und komplexe Messanordnungen erforderlich. Ferner sind sie auf spezifische Parameter fokussiert und auf wenige Messorte eingeschränkt, wie z. B. das Ohr. Zur Messung verschiedener Parameter eines Körpergewebes müssen daher unterschiedliche Messsysteme gleichzeitig oder nach einander verwendet werden. Zudem können auch die bekannten nicht-invasiven Methoden für den Patienten unangenehm und riskant sein.

Aus US 2013/072807 A1 ist ein Überwachungssystem für eine Person bekannt, welches unter anderem auch eingerichtet ist, um aus nichtinvasiv ermittelbaren Konzentrationswerten von Blutparametern auf den Blutdruck der Person zu schliessen.

Aus US 2004/054290 A ist eine optische Koppelvorrichtung bekannt zur nicht-invasiven Überwachung eines Bereichs eines lebenden Gewebes, wobei Strahlung mit einem sich zeitlich verändernden Muster von einer Quellensonde in das Gewebe emittiert und von einer Detektiersonde detektiert wird.

Es ist daher eine Aufgabe der vorliegenden Erfindung ein Messsystem und ein Verfahren zur Verfügung zu stellen, mit welchen eine einfache und schnelle Ermittlung von Parametern eines Körpergewebes möglich ist, die mit wenigen Systemkomponenten auskommen, vorzugweise mehrere verschiedene Parameter gleichzeitig erfassen können und eine für einen Patienten angenehme Anwendung erlauben.

Diese Aufgabe wird von der Erfindung durch ein Messsystem nach Anspruch 1 und ein Verfahren nach Anspruch 12 gelöst. Vorteilhafte Ausgestaltungen und weitere Ausführungsbeispiele sind in den abhängigen Ansprüchen beschrieben.

Die vorliegende Erfindung schlägt ein Messsystem zur Bestimmung wenigstens eines Druckparameters in einem Körpergewebe im Gehirn oder im Muskel vor, das wenigstens eine Messvorrichtung zum Anbringen am Körper, eine optische Einheit zur Emission und Detektion von mindestens vier Lichtwellen mit unterschiedlichen Wellenlängen, wobei wenigstens eine der Wellenlängen der Lichtwellen im Bereich der Absorption eines Konstituenten des Körpergewebes liegt, wenigstens einen Lichtleiter zwischen optischer Einheit und Messvorrichtung zur Übermittlung von Lichtwellen mit unterschiedlicher Wellenlänge und eine Auswerteeinheit zur Auswertung von Messwellensignalen, welche von der Messvorrichtung empfangen und übermittelt werden, umfasst. Von der optischen Einheit emittierte Lichtwellen werden mittels der Messvorrichtung in ein optisches Messvolumen im Körpergewebe eingestrahlt. Von der Messvorrichtung aus dem Messvolumen empfangene Messwellensignale werden an die Auswerteeinheit übermittelt. Somit kann mittels Bestimmung der Lichtabsorption einer Wellenlänge im Messvolumen des Körpergewebes der Konzentrationswert des Konstituenten bestimmt werden.

Nach der Erfindung ist die Auswerteeinheit dazu ausgelegt, empfangene Messwellensignale, basierend auf der Absorption der emittierten Lichtwellen, aufzubereiten um eine Pulsatilität eines im Messvolumen gemessenen Konzentrationsparameters zu bestimmen, und die Auswerteeinheit umfasst einen Transformationsalgorithmus, um aus der Pulsatilität des Konzentrationsparameters einen Druckparameter in dem Körpergewebe zu bestimmen, durch eine Vergleichsoperation der Änderung bzw. des Verlaufs einer Amplitude der Pulsatilität mit einer Referenz-Druck-Volumenkurve.

Mit dem Messsystem nach der Erfindung ist es somit möglich, mehrere verschiedene Parameter eines Körpergewebes durch eine einfache Absorptionsmessung, wie sie im Wesentlichen bekannt ist, zu erhalten. Die Messergebnisse der Absorptionsmessung zur Ermittlung einer Konzentration eines Konstituenten werden durch die Auswerteeinheit weiter verarbeitet, indem charakteristische Eigenschaften der Pulsatilität bestimmt werden. Der ermittelten Pulsatilität wird mittels des Transformationsalgorithmus entsprechend der jeweiligen charakteristischen Eigenschaften ein Druckparameter zugeordnet, der den Druck im Körpergewebe angibt. So kann ein Druckparameter des Körpergewebes ermittelt werden, ohne zusätzliche Druckmessgeräte zu verwenden. Zudem kann die Druckbestimmung gleichzeitig mit der Bestimmung anderer Körperparameter erfolgen.

Gemäss einem Beispiel der Erfindung ist das Messsystem zur Bestimmung von Konzentrationswerten wenigstens eines Konstituenten ausgebildet. Der Transformationsalgorithmus transformiert in diesem Fall die Pulsatilität der im Messvolumen gemessenen Konzentrationswerte des Konstituenten des Körpergewebes in den Parameter des Drucks im Körpergewebe. Vorzugsweise wird von dem Messsystem im Messvolumen eine Konzentration an oxygeniertem Hämoglobin, desoxygeniertem Hämoglobin und/oder eine Gesamthämoglobinkonzentration ermittelt. Die Pulsatilität dieser gemessenen Konzentration wird dann in den Parameter des Drucks in dem Körpergewebe transformiert.

Gemäss der Erfindung ist das Messsystem zur Bestimmung eines Konstituenten in einem Messvolumen im Gehirn ausgebildet. In einer Ausführungsform kann mittels des Transformationsalgorithmus aus der Pulsatilität der Konzentration von Konstituenten im Gehirnmessvolumen ein Parameter des intrakraniellen Drucks bestimmt werden.

Gemäss der Erfindung ist das Messsystem zur Bestimmung eines Konstituenten in einem Messvolumen in einem Muskel ausgebildet. In einer Ausführungsform kann mittels des Transformationsalgorithmus aus der Pulsatilität der Konzentration von Konstituenten im Muskelmessvolumen ein kompartimentaler Druckparameter des Muskels bestimmt werden.

Der Erfindung liegt die überraschende Erkenntnis zugrunde, dass aus einer Kombination der Lehre gemäss der Monro Kellie Doktrin und dem Verlauf der Pulsatilität in einfacher Weise auf verschiedene zerebrale Parameter geschlossen werden kann. Insbesondere können der intrakranielle Druck, die Konzentration verschiedener Hirnkonstituenten und das Hirnwasservolumen bestimmt werden. Weiter können auch Metaboliten und Enzyme bestimmt werden. Diese Erkenntnis lässt sich zudem auch auf andere Organe übertragen. Insbesondere konnte auch für die Bestimmung eines Druckparameters im Muskel ein Transformationsalgorithmus entwickelt werden. Ferner kann das Messsystem auch zur Bestimmung von Parametern im Knochen angewendet werden.

Die der Erfindung zugrunde liegenden Sachverhalte werden am Beispiel einer Messung am Gehirn näher erläutert. Der Schädel kann in erster Näherung als unelastisch angenommen werden. Volumenänderungen innerhalb des Schädels bedingt durch die Differenz von einfliessendem und ausfliessendem Blut bewegen sich regulär im Rahmen von 1 ml. Alle Substanzen innerhalb des Schädels können als inkompressibel angenommen werden. Ferner kann eine Volumenzunahme eines Konstituenten des Gehirns, z. B. eine Gewebevolumenzunahme durch ein Hirnödem, nur durch die kompensatorische Abnahme eines anderen Konstituenten, z. B. Abnahme des Hirnwassers oder des Blutvolumens, ausgeglichen werden. Nach Aufbrauchen dieser Reserveräume kommt es zu einem Anstieg des intrakraniellen Drucks. Man spricht hier von der Compliance des Gehirns. Obgleich das intrakranielle Druck-Volumen Verhältnis von solchen Kompensationsmechanismen abhängig ist, verhält es sich zumindest für ein Individuum annähernd konstant. Unter pathologischen Bedingungen sind Masseneffekte, wie etwa eine Hirnschwellung, Blutung, Tumorbildung, etc., jedoch relevanter und führen durch die Volumenzunahme zum Anstieg des intrakraniellen Drucks. Eine Druck-Volumen-Kurve ist monoexponentiell und kann in bekannter Weise empirisch ermittelt werden.

Andererseits haben die beschriebenen pathologischen Zustände Auswirkungen auf den Verlauf der Pulsatilität des Blutflusses. Bei erhöhtem intrakraniellem Druck, beispielsweise bei einer Hirnschwellung, muss das Herz zunehmend gegen den intrakraniellen Druck Blut in den Schädel pumpen. Gleichzeitig nimmt bei Verengung der Gefässe die Geschwindigkeit des Blutes in den Gefässen zu. Die Pulswelle des Blutflusses induziert im Schädel eine Volumenänderung und nimmt somit Einfluss auf den intrakraniellen Druck und die Compliance.

Es wurde nun festgestellt, dass aus dem Verlauf, bzw. aus der Veränderung der Amplitude der Pulsatilität eines Blutparameters mittels der Druck-Volumen-Kurve auf den intrakraniellen Druck geschlossen werden kann. Insbesondere kann aus dem Verlauf der Amplitude der Blutpulsatilität der Hämoglobingehalt in einem Hirnsegment und somit der intrakranielle Druck ermittelt werden. Auf Grund der oben erwähnten Effekte verändert sich die ICP-Kurven-Konfiguration und somit die Morphologie der ICP-Kurve.

Gemäss einer Ausführungsform der Erfindung werden mittels optischer Messverfahren der Verlauf, bzw. die Änderung beispielsweise von Konzentrationswerten der verschiedenen Konstituenten eines Körpergewebes in Form einer Messwelle, bzw. eines optischen Messsignals für Gewebewasser, oxygeniertes Hämoglobin, desoxygeniertes Hämoglobin und vorzugsweise auch für den Gesamthämoglobinwert erfasst. Aus einer Pulsatilität der Messwellensignale für die verschiedenen Konstituenten wird eine Pulsatilität von Werten des Druckparameters, wie z. B. dem intrakraniellen Druck, ermittelt. Aus der somit gewonnenen Pulsatilität der intrakraniellen Druckwerte wird die Compliance und der mittlere intrakranielle Druck bestimmt.

Die Pulsatilität einer Konzentration von Konstitutenten wird z. B. durch die Peaks innerhalb eines Pulses charakterisiert, wie genauer in der Figurenbeschreibung dargelegt wird. Im Allgemeinen nimmt die Amplitude bei ansteigendem intrakraniellem Druck ab, sobald die Kompensationsmechanismen ausgeschöpft sind.

Die Pulsatilität des Blutflusses und somit der Verlauf, bzw. Veränderung, deren Amplitude kann in einfacher Weise durch ein optisches Messsystem nach der Erfindung mit einer Messvorrichtung zur nicht-invasiven Messung von Parametern eines Körpergewebes ermittelt werden. Die Messvorrichtung umfasst dabei ein auf ein Organ, wie etwa die Kopfhaut oder die Haut über einem Muskel, aufzubringendes Emitter-Detektor-System, das zur Emission und Detektion der verschiedenen Lichtwellen vorgesehen ist. Eine solche Messvorrichtung ist z. B. in der Schweizer Patentanmeldung der Anmelderin mit dem Titel "Messvorrichtung zur Bestimmung zerebraler Parameter", Nr. 02266/12 vom 06.11.2012, beschrieben. Eine solche Messvorrichtung mit einer Sensoreinheit und einer Sensormatte ist zum lösbaren Aufbringen der Messvorrichtung auf einer Körperoberfläche, wie z. B. der Schädeloberfläche, etwa auf der Stirn, vorgesehen. Die Ausführungen in Bezug auf Merkmale dieser Messvorrichtung, sowie deren Vorteile zur Durchführung von Messungen am Körpergewebe, insbesondere am Gehirngewebe, werden vollumfänglich in die Beschreibung der vorliegenden Erfindung einbezogen. Insbesondere wird auf die Ausführungen in Bezug auf optische Messungen von Parametern Bezug genommen.

Ferner können mit Hilfe einer solchen Messvorrichtung Volumina innerhalb des Schädels ermittelt werden und somit Absolutwerte für die Konzentration von Wasser und Hämoglobin im Hirngewebe ermittelt werden. Blut setzt sich aus Blutplasma (Wasser, Eiweisse, etc.) und korpuskulären Bestandteilen, darunter Erythocyten mit Hämoglobin zusammen. Die Konzentration der Erythocyten bestimmt den Hämatokrit und somit auch die Massenkonzentration von Wasser im Blut. Dabei wird das zerebrale Blutvolumen durch das Verhältnis von Masse des Gehirns zu Blutvolumen und durch das Verhältnis von Hämatokrit zur Hämoglobinkonzentration im Gehirn bestimmt. Dabei besteht das intrakranielle Volumen aus den Volumina weisser und grauer Hirnsubstanzen, dem Blutvolumen und dem Liquorraum (Hirnwasser). Gleiches gilt für eine Messung am Muskel, wobei sich das Volumen des Muskels histologisch aus Muskelzellen mit Myofribrillen zusammensetzt. Chemisch enthalten diese rund ¾ Wasser und ¼ feste Stoffe. Die Feststoffbestandteile sind ca. 20% Proteine, vor allem Myosin, Lipide, Glykogen, Kreatin, etc..

Das Messsystem kann einen Volumenmesswert ermitteln, der sich aus dem intrakraniellen Volumen und extrazerebralen Anteilen, wie dem Liquorvolumen, Knochenvolumen und dem Hautvolumen, zusammensetzt.

Nach der Erfindung wird das Messsystem vorzugsweise bei jeder Messung am gleichen Messort eines Patienten angeordnet, so dass bei jeder Messung dasselbe optische Volumensegment vermessen wird. Der extrakranielle Anteil, bzw. der externe Anteil beim Muskel, kann somit über die Zeit als konstant angesehen werden. Es hat sich nun gezeigt, dass die mit dem Messsystem gemessene Konzentration eines vermessenen Volumens für den gesamten Schädel, bzw. Muskel, repräsentativ ist. Insbesondere ist ein mit dem Messsystem im Nahinfrarot-Spektroskopieverfahren (NIRS) vermessenes Volumen annäherungsweise für den gesamten Schädel, bzw. Muskel, repräsentativ. Mit dem Messsystem kann somit ein optisches Volumensegment des Körpergewebes vermessen werden und daraus auf die Zusammensetzung der Gewebeanteile im gesamten Hirn oder Muskel geschlossen werden. Dabei wird die Konzentrationsänderung im durchstrahlten Volumensegment ermittelt. Das Gesamtvolumen kann, falls erforderlich, durch bekannte Messverfahren, wie etwa CT-Verfahren, erfasst werden. Die Transformation der mit der Messvorrichtung ermittelten Konzentrationswerte in Volumenwerte erfolgt mittels der Auswertevorrichtung, wie etwa einem Computer, die an die Messvorrichtung angeschlossen ist oder der die Messwerte zugeführt werden können. Die Messwerte können als optische Signale, wie den von der Messvorrichtung empfangenen Messwellen, oder als elektrische Signale übermittelt werden.

Mit einem Messsystem und einem Verfahren nach der vorliegenden Erfindung ist es auch in einfacher, vorzugsweise nicht-invasiver Weise möglich, eine Gewebewasserbestimmung durchzuführen. Dabei werden von einer Lichtquelle des Messsystems mehrere Lichtwellen unterschiedlicher Wellenlängen emittiert und durch die Messvorrichtung in das optische Volumensegment geleitet, wobei die Lichtwellen vorzugsweise am Rand des sogenannten biooptischen Fensters liegen. Wenigstens eine Wellenlänge liegt im Bereich der Wasserabsorption und ist für die Bestimmung der Wasserkonzentration geeignet und charakteristisch. Mittels weiterer Wellenlängen kann oxygeniertes und desoxygeniertes Blut sowie Gewebematerial vermessen werden.

Im Allgemeinen können mit einem Messsystem und einem Verfahren nach der vorliegenden Erfindung verschiedene Effekte gleichzeitig gemessen werden. Es kann der Abstand zwischen Hirn und Schädel, bzw. dessen Änderung, durch eine transkranielle Messung auf Basis einer NIRS-Messung ermittelt werden. Bei zunehmendem Druck kann die Abnahme des zugeführten Blutvolumens gemessen werden, durch die Änderung, bzw. den Verlauf der Amplitude der Pulsatilität, wie oben erwähnt. Bei der Verwendung von mehreren verschiedenen Wellenlängen, die im Wesentlichen simultan ein optisches Volumenmesssegment des Körperorgans, wie z. B. des Schädels, durchdringen, kann die Konzentration verschiedener Konstituenten, z. B. Hirnkonstituenten, ermittelt werden, für welche deren charakteristische optische Spektren bekannt sind. Dabei ist es vorteilhaft, wenn das Gewicht der verschiedenen Absorptionen vergleichbar ist, d. h. das Produkt von Absorptionskoeffizient und der zu messenden Konzentration soll für alle zu messenden Konstituenten des Gewebes innerhalb der gleichen Grössenordnung sein. Dadurch wird eine repräsentative Messgenauigkeit erreicht.

Gemäss der Erfindung weist das optische Messsystem eine Messvorrichtung zur Vermessung eines optischen Volumensegments des Körpergewebes auf, die von einer Lichtquelle mit Licht mehrerer verschiedener Wellenlängen versorgt wird. Die Lichtquelle stellt Lichtstrahlen mit wenigstens vier verschiedenen Wellenlängen zur Verfügung. Wenigstens eine Wellenlänge liegt im Bereich der Wasserabsorption. Die weiteren Wellenlängen werden auf die übrigen Gewebekonstituenten, z. B. Hirnkonstituenten, abgestimmt. Beispielsweise werden Wellenlängen zwischen 750nm und 1100nm verwendet. Dadurch kann z. B. die Hämoglobinkonzentration und die Konzentration weisser und grauer Substanzen gemessen werden. Bei einer Messung am Muskel werden die Wellenlängen vorzugsweise auf die Absorptionsspektren von Proteinen und auch von Myoglobin abgestimmt. Proteine haben beispielsweise optisch messbare Reaktionen bei 200-300nm je nach Grösse. Ferner werden die verschiedenen Wellenlängen derart gewählt, dass das Produkt von Absorptionskoeffizient und Konzentration für alle zu messenden Konstituenten innerhalb der gleichen Grössenordnung ist. Die Messung kann im Wesentlichen analog einem NIRS-Verfahren erfolgen.

Weiter weist das Messsystem eine Auswertevorrichtung auf, auf der z. B. ein numerisches Verfahren implementiert ist, das mittels eines linearen Gleichungssystems die Konzentration der Konstituenten im Gewebe aus der optischen Messung ermittelt. Für das Gleichungssystem kann z. B. der allgemein bekannte Verlauf der Druck-Volumen Kurve des zerebralen oder muskulären Drucks verwendet werden, welche z. B. durch eine übliche Messreihe ermittelt werden kann. Oder es kann eine Durchschnittskurve als Referenz verwendet werden. Aus der Veränderung der Pulsatilität entlang dieser Kurve kann auf den gesuchten Parameter, insbesondere den Druck, geschlossen werden. Hierfür kann z. B. die Pulsatilität bei zwei unterschiedlichen Druck-, bzw. Volumenwerten, ermittelt und über diese Messwerte extrapoliert werden.

Aus der Bestimmung der Konzentration der einzelnen Konstituenten im Hirngewebe kann somit ein Ansteigen oder Abfallen der Hirnwasserkonzentration abgeleitet werden. Auf diese Weise kann z. B. eine Zunahme oder Abnahme eines Hirnödems bestimmt werden.

Weiter ist es vorteilhaft, dass das Messsystem mit wenigstens einer Wellenlänge, die auf Veränderungen der Blutkonzentration reagiert, eine Pulsatilität dieses Parameters messen kann. Hierfür ermittelt z. B. die oben genannte Messvorrichtung das im Messvolumensegment gestreute oder reflektierte Licht. Das Signal des detektierten Lichtstrahls, d. h. das empfangene Messwellensignal, wird in die zugehörige Pulsatilität des gemessenen Konzentrationswerts von Konstitutenten übersetzt. Vorzugsweise wird die Pulsatilität an Hand mehrerer verschiedener Wellenlängen, die auf Veränderungen der Blutkonzentration reagieren, ermittelt und ein Mittelwert aus diesen Informationen gebildet. Bei gleichbleibenden Bedingungen bleibt die Amplitude der Pulsatilität gemittelt über mehrere Pulswellen in etwa gleich. Verändert sich die Amplitude weist dies auf eine Veränderung des Drucks hin.

Das Messsystem nach der vorliegenden Erfindung kann auch mit zwei oder mehr Messvorrichtungen der oben genannten Art verwendet werden, die jeweils Messsignale an die Auswerteeinheit liefern. Die Messvorrichtungen können z. B. an sich gegenüberliegenden Bereichen des Schädels oder eines Muskels vorgesehen sein. Durch mehrere Messvorrichtungen können verschiedene Hirnareale, z. B. bezüglich eines unterschiedlichen Hirnwassergehalts oder allgemein bezüglich der Zusammensetzung des Gewebes überwacht werden.

Das Messsystem kann auch eine Markervorrichtung umfassen, mittels der ein Farbstoff, vorzugsweise ein indocyaningrüner (ICG) Farbstoff, in den Blutfluss eingebracht werden kann. Das Messsystem kann dann in einem ICG-dye dilution Modus verwendet werden, mit dem das Blutvolumen im Gewebe bestimmt werden kann. Diese Messgrösse wird mit einer kontinuierlich zu messenden Grösse, wie z. B. dem totalen Haemoglobin, gekoppelt, wodurch Veränderungen im Blutvolumen detektiert werden können.

Alternativ zu einer nicht-invasiven Messvorrichtung der oben beschriebenen Art, kann das Prinzip der vorliegenden Erfindung unter Einsatz einer herkömmlichen Sonde, vorzugsweise einer minimal-invasiven Sonde, z. B. in Form eines intrakraniellen Katheters, vorteilhaft eingesetzt werden. Die Ermittlung der gewünschten Parameter kann mittels der empfangenen Messsignale der Sonde gemäss der vorliegenden Erfindung erfolgen.

Die Erfindung wurde an Hand mehrerer Ausführungsformen dargestellt. Die einzelnen technischen Merkmale einer Ausführungsform können durchaus auch in Kombination mit einer anderen Ausführungsform mit den dargelegten Vorteilen verwendet werden. Die Beschreibung der erfindungsgemässen technischen Merkmale ist daher nicht auf die jeweilige Ausführungsform beschränkt. Insbesondere können die Merkmale einer Messung am Schädel auf eine Messung am Muskel oder Knochen übertragen werden.

Eine vorteilhafte Ausführungsform der Erfindung wird im Folgenden anhand der Zeichnung dargestellt, die lediglich zur Erläuterung dient und nicht einschränkend auszulegen ist. Aus der Zeichnung offenbar werdende Merkmale der Erfindung sollen als zur Offenbarung der Erfindung gehörend betrachtet werden. In den Zeichnungen zeigen:
Fig. 1 eine Druck-Volumen Kurve des intrakraniellen Drucks und des intrakraniellen Volumens,
Fig. 2 eine schematische Übersicht einer Zuordnung verschiedener Pulsatilitätsergebnisse zu Parametern des intrakraniellen Drucks und
Fig. 3 eine schematische Darstellung eines Messsystems nach der vorliegenden Erfindung.

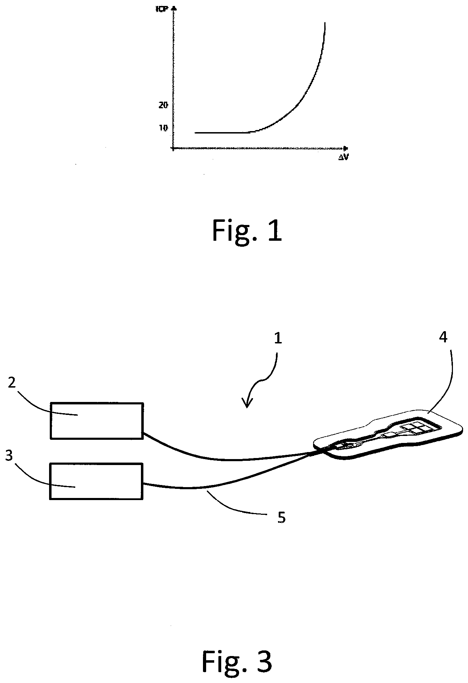

In Figur 1 ist an Hand einer Druck-Volumen Kurve der Zusammenhang zwischen dem intrakraniellen Druck und dem intrakraniellen Volumen gezeigt. Dabei ist der Druck in Torr [mmHg] und das Volumen in Einheiten einer Druckveränderung ΔV angegeben. Daraus ist ersichtlich, dass in einem niederen Bereich des intrakraniellen Drucks bis ca. 10 mmHg eine Volumenerhöhung keine wesentliche Druckerhöhung hervorruft. In diesem Bereich können die Kompensationsmechanismen eine Volumenänderung ausgleichen, ohne dass der Druck ansteigt. Im einem noch normalen Bereich zwischen 10 mmHg und 20 mmHg beginnt der intrakranielle Druck mit einer Volumenerhöhung anzusteigen. Ab einem Druck von 20 mmHg, das einem pathologischen Druckbereich entspricht, ruft eine nur geringe Volumenänderung eine starke Erhöhung des intrakraniellen Drucks hervor. Dieser Zusammenhang zwischen intrakraniellem Druck und intrakraniellem Volumen wird bei dem Messsystem und -verfahren nach der vorliegenden Erfindung zur Ermittlung der zerebralen Parameter verwendet, wie vorher beschrieben.

Entlang dieser Kurve zeigt eine Pulsatilität von Blutwerten, wie z. B. die Werte von oxygeniertem oder desoxygeniertem Blut, eine Veränderung, bzw. einen charakteristischen Verlauf. Durch die Ermittlung der Pulsatilität und die Druck-Volumen Kurve kann somit auf den intrakraniellen Druck geschlossen werden. Ferner können die Konzentrationen der unterschiedlichen Konstituenten des Gehirns ermittelt werden.

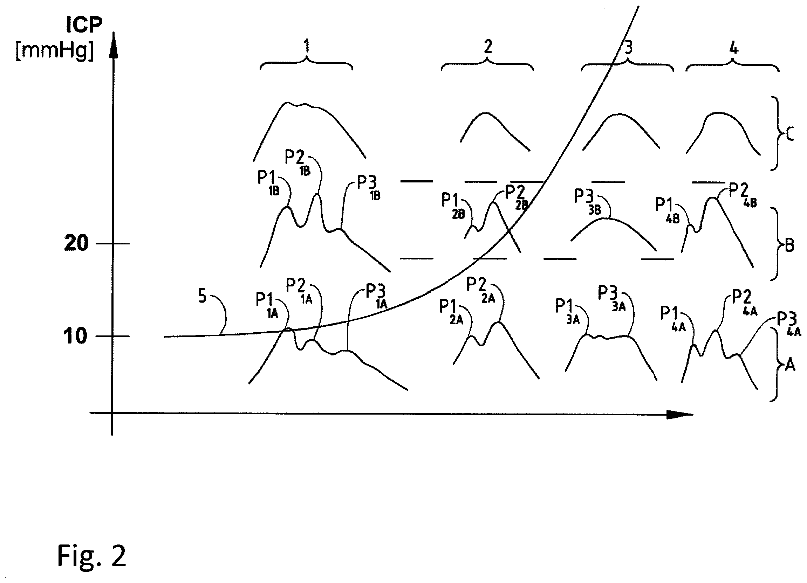

In Figur 2 ist für eine Untersuchung des Gehirns eine schematische Übersicht einer Zuordnung verschiedener Pulsatilitätsergebnisse zu Parametern des intrakraniellen Drucks dargestellt. Auf einer vertikalen Achse ist wiederum der intrakranielle Druck in Torr [mmHg] gezeigt. In Spalte 1 ist die Morphologie eines Pulses einer ICP Messkurve bei drei unterschiedlichen Werten des intrakraniellen Drucks gezeigt. In Spalte 2 ist der Pulsverlauf einer Konzentration von oxygeniertem Hämoglobin (Hb_{oxy}), in Spalte 3 der Pulsverlauf einer Konzentration von desoxygeniertem Hämoglobin (Hb_{deoxy}) und in Spalte 4 der Pulsverlauf einer Gesamthämoglobinkonzentration (Hbtot) jeweils bei drei unterschiedlichen Werten des intrakraniellen Drucks gezeigt. Die drei unterschiedlichen Werte des intrakraniellen Drucks werden in Zeilen A, B und C aufgeteilt. Der Pulsverlauf in Zeile A entspricht einem intrakraniellen Druck von ca. 10 mmHg, in Zeile B einem intrakraniellen Druck von ca. 20 mmHg und in Zeile C einem intrakraniellen Druck von deutlich über 20 mmHg.

Der Verlauf der Pulsatilität des intrakraniellen Druck ist bekannt und wird z. B. beschrieben in "American Journal of Critical Care", 2008;17:545-554 - Intracranial Pressure Waveform Morphology and Intracranial Adaptive Capacity". Unter normalen Bedingungen, wie bei einem Pulsverlauf in Spalte 1, Zeile A, weist der Pulsverlauf drei Peaks auf. Peak P1_{1A} stammt aus der Pulsation des choroidalen Plexus (percussion wave) mit einer annähernd konstanten Amplitude. Peak P2_{1A} stammt aus einem Rückstoss nach dem ersten arteriellen Ausschlag (tidal wave) und variiert in Form und Amplitude. Peak P3_{1A} entstammt einer venösen Reaktion nach einer dikroten Senke (dicrotic wave). Spalte 1, Zeile B zeigt den Pulsverlauf eines ICP Pulses mit im Vergleich zu Zeile A erhöhtem ICP-Wert. Peak P2_{1B} ist deutlich erhöht, während Peak P1_{1B} im Wesentlichen gleich bleibt. Spalte 1, Zeile C zeigt den Pulsverlauf eines ICP Pulses im pathologischem Druckbereich über 20 mmHg. Der Pulsverlauf ist abgeflacht und zeigt nur gering ausgebildete Peaks. Im Gegensatz zur vorliegenden Erfindung wird in der oben genannten Publikation dargelegt, dass die Peaks des Pulsverlaufs des intrakraniellen Drucks kein klinisch relevanter Faktor seien und sich nicht zur Bestimmung eines Zustands eines Patienten eigneten.

Der Pulsverlauf einer Konzentration von oxygeniertem Hämoglobin, desoxygeniertem Hämoglobin und einer Gesamthämoglobinkonzentration wurde mittels einer Messvorrichtung gemäss Figur 3 durch NIRS-Messung ermittelt. Der Pulsverlauf der Konzentration von Hb_{oxy} unter normalen Bedingungen ist in Spalte 2, Zeile A gezeigt. Peak P1_{2A} zeigt eine Amplitude des choroidalen Plexus und Peak P2_{2A} eine Amplitude einer arteriellen Komponente des Pulses. Die arterielle Komponente steigt mit steigendem intrakraniellem Druck deutlich an, wie in Spalte 2, Zeile B, an der Erhöhung des Peak P2_{2B} zu sehen ist. Der Pulsverlauf der Konzentration von Hb_{desoxy} unter normalen Bedingungen ist in Spalte 3, Zeile A gezeigt. Peak P1_{3A} zeigt wiederum die Amplitude des choroidalen Plexus. Die arterielle Komponente des Pulses ist kaum ersichtlich, wohingegen die venöse Komponente den Pulsverlauf stark prägt mit einem Peak P3_{3A}. Bei erhöhtem Druck dominiert die venöse Komponente den Pulsverlauf mit Peak P3_{3B}, wie in Spalte 3, Zeile B ersichtlich ist. Der Pulsverlauf der Gesamthämoglobinkonzentration weist im normalen Druckbereich deutlich ausgeprägte Peaks P1_{4A}, P2_{4A} und P3_{4A} auf, siehe Spalte 4, Zeile A. Bei erhöhtem Druck verschwindet die venöse Komponente und die arterielle Komponente ist ausgeprägter mit Peak P2_{4B}. Im im pathologischem Druckbereich über 20 mmHg zeigt der Pulsverlauf keine einzelnen Peaks mehr, vielmehr wird ein Puls durch die Breite eines einzigen Maximums bestimmt.

Messungen haben gezeigt, dass der Verlauf der Pulsatilität der mittels der Messvorrichtung durch Absorptionsmessung bestimmten Konzentrationswerte von Konstituenten, reproduzierbar ist und den Werten des intrakraniellen Drucks eindeutig zugeordnet werden kann. Grundsätzlich ist hierfür ein Konstituent ausreichend, wie z. B. oxygeniertes Hämoglobin. Die Verwendung mehrerer Konstituenten, z. B. Hb_{oxy}, Hb_{deoxy} und Hbₜₒₜ kann jedoch die Messgenauigkeit erhöhen. Der Transformationsalgorithmus des Messsystems umfasst hierfür einen Vergleichsalgorithmus, womit der gemessene Pulsatilitätsverlauf einem bestimmten Druckwert zugeordnet werden kann. Hierfür kann auf Referenzkurven für den Verlauf des intrakraniellen Drucks zurückgegriffen werden. Darüber hinaus kann die Pulsatilität der einzelnen Konzentrationen von Konstituenten mittels der Druck-Volumen-Kurve aus Figur 1 einem intrakraniellen Druck zugeordnet werden. Auch eine Kombination von einem Vergleich mit den Peaks des intrakraniellen Drucks ist möglich, um den anliegenden Druck zu bestimmen.

Vorteilhaft ist es, dass bei der Erfindung die Bestimmung von Konzentrationswerten von Konstitutenten verwendet werden kann, die in einfacher Weise ermittelt werden können und für sich genommen für die Überwachung eines Patienten hilfreich sind. Es ist nicht erforderlich für die Bestimmung des Drucks eine zusätzlich Messeinheit zu verwenden.

In Figur 3 ist ein Messsystem 1 zur Messung wenigstens eines Körperparameters nach der Erfindung schematisch dargestellt. Das Messsystem umfasst wenigstens eine Messvorrichtung 4, eine Licht emittierende optische Einheit 3 und eine Auswerteeinheit 2. Die Messvorrichtung ist als nicht-invasiver Patch ausgebildet, der auf einem Körperorgan, wie z. B. an einem Schädel oder auf einem Muskel, über einem optischen Messvolumen im Körpergewebe vorgesehen wird. Weiter sind Lichtleiter 5 zwischen optischer Einheit 3, Messvorrichtung 4 und Auswerteeinheit 2 zur Übermittlung von Lichtwellen vorgesehen sind. Dabei werden von der optischen Einheit 3 emittierte Lichtwellen mittels der Messvorrichtung 4 in das optische Messvolumen im Gehirn eingestrahlt und aus dem Gehirn von der Messvorrichtung 4 empfangene Messwellen an die Auswerteeinheit 2 übermittelt. Die optische Einheit 3 stellt mehrere Lichtwellen unterschiedlicher Wellenlänge an die wenigstens eine Messvorrichtung 4 zur Verfügung. Dabei liegt vorzugsweise wenigstens eine Wellenlänge im Bereich der Wasserabsorption und jede weitere Wellenlänge ist entsprechend der Absorption eines zu bestimmenden Körperparameters gewählt.

Erfindungsgemäss sind wenigstens vier Lichtwellen unterschiedlicher Wellenlänge vorgesehen, wobei das Gewicht der verschiedenen Absorptionen der Wellenlängen im optischen Messvolumen vorzugsweise innerhalb einer Grössenordnung liegt. Für jeden Konstituenten im Körpergewebe ist vorteilhafter Weise eine Wellenlänge entsprechend der Absorption zur Messung einer Konzentration dieses Konstituenten vorgesehen. Vorzugsweise ist eine Wellenlänge entsprechend der Absorption zur Messung einer Konzentration eines Konstituenten im Körpergewebe, eine weitere Wellenlänge entsprechend der Absorption zur Messung einer Konzentration von desoxygeniertem Blut und, falls gewünscht, noch eine weitere Wellenlänge entsprechend der Absorption zur Messung einer Konzentration von oxygeniertem Blut vorgesehen. Die Lichtwellen und die Messwellen können für alle zu messenden Parameter simultan durch die Messvorrichtung geführt werden.

Das System kann in den entsprechenden Wellenlängen, die auf Veränderungen der Blutkonzentration reagieren, die Pulsatilität des Messwertes messen. Bei gleichbleibenden Bedingungen sollte die Amplitude der Pulsatilität gemittelt über mehrere Pulswellen in etwa gleich bleiben. Verändert sich die Amplitude weisst dies auf eine Veränderung des Drucks im Körpergewebe hin. Aus den gemessenen Werten wird mittels einer entsprechenden Korrelation in der Auswerteeinheit eindeutig ein Anstieg, respektive ein Abfall des Drucks bestimmt. Aus der Bestimmung der Konzentration der Konstituenten im Körpergewebe wird ein Anstieg oder Abfall der Gewebewasserkonzentration detektiert. Damit lässt sich eine Zu- oder Abnahme eines Ödems bestimmen. Vorzugsweise wird mit dem Messsystem gleichzeitig die Hämodynamik, die Konzentration der Gewebebestandteile, die Stärke des Ödems und des Drucks bestimmt.

Ein Beispiel für den Einsatz des erfindungsgemässen Messsystems ist die Untersuchung des Gehirns und die Ermittlung des intrakraniellen Drucks, bzw. dessen Veränderung, wie vorher dargelegt. Das Messsystem und das Verfahren sind auch zur Untersuchung von Muskelgewebe geeignet, um den Druck in einem Muskelkompartment, bzw. dessen Veränderung zu erfassen.

## Patentansprüche

1. Messsystem zur Bestimmung wenigstens eines Druckparameters in einem Körpergewebe in einem Gehirn oder einem Muskel umfassend:
- wenigstens eine Messvorrichtung zum Anbringen am Körper,
- eine optische Einheit zur Emission und Detektion von mindestens vier Lichtwellen mit unterschiedlichen Wellenlängen, wobei wenigstens eine der Wellenlängen der Lichtwellen im Bereich der Absorption eines Konstituenten des Körpergewebes liegt,
- wenigstens einen Lichtleiter zwischen optischer Einheit und der wenigstens einen Messvorrichtung zur Übermittlung von den Lichtwellen mit unterschiedlicher Wellenlänge, und
- eine Auswerteeinheit zur Auswertung von Messwellensignalen, welche von der Messvorrichtung empfangen und übermittelt werden,
- wobei von der optischen Einheit emittierte Lichtwellen mit unterschiedlichen Wellenlängen mittels der wenigstens einen Messvorrichtung in ein optisches Messvolumen in dem Körpergewebe einstrahlbar sind und die aus dem optischen Messvolumen von der Messvorrichtung empfangenen Messwellensignale an die Auswerteeinheit übermittelbar sind,
wobei
- die Auswerteeinheit dazu ausgelegt ist, die empfangenen Messwellensignale, basierend auf der Absorption der emitiierten Lichtwellen, aufzubereiten,
- um eine Pulsatilität eines im Messvolumen gemessenen Konzentrationsparameters zu bestimmen, und wobei
- die Auswerteeinheit einen Transformationsalgorithmus umfasst, der dazu ausgelegt ist, aus der Pulsatilität des Konzentrationsparameters einen Druckparameter in dem Körpergewebe zu bestimmen, durch eine Vergleichsoperation der Veränderung bzw. des Verlaufs einer Amplitude der Pulsatilität mit einer Referenz-Druck-Volumenkurve.

2. Messsystem nach Anspruch 1, **dadurch gekennzeichnet, dass** wenigstens einer der Konzentrationswerte der Konstituenten eine Konzentration an oxygeniertem Hämoglobin, desoxygeniertem Hämoglobin und/oder einer Gesamthämoglobinkonzentration ist.

3. Messsystem nach Anspruch 1 oder 2, **dadurch gekennzeichnet, dass** der Druckparameter ein Parameter des intrakraniellen Drucks im Gehirn ist.

4. Messsystem nach Anspruch 1 oder 2, **dadurch gekennzeichnet, dass** der Druckparameter ein kompartimentaler Druck des Muskels ist.

5. Messsystem nach einem der vorhergehenden Ansprüche, **dadurch gekennzeichnet, dass** eine der Wellenlängen der emittierten Lichtwellen im Bereich der Wasserabsorption liegt und eine weitere der Wellenlängen im Bereich der Absorption eines der Konstiutenten im Körpergewebe gewählt ist.

6. Messsystem nach einem der vorhergehenden Ansprüche, **dadurch gekennzeichnet, dass** wenigstens vier Lichtwellen mit unterschiedlichen Wellenlängen von der optischen Lichteinheit emittierbar sind zur Bestimmung von vier Konstituenten in dem Körpergewebe, wobei ein Produkt von Absorptionskoeffizient und Konzentrationswert des Konstituenten für die vier Konstituenten in dem Körpergewebe innerhalb einer Grössenordnung liegt.

7. Messsystem nach einem der vorhergehenden Ansprüche, **dadurch gekennzeichnet, dass** für unterschiedliche Konstituenten im Körpergewebe eine Wellenlänge entsprechend der Absorption durch einen der Konstituenten zur Messung einer Konzentration dieses Konstituenten vorgesehen ist, vorzugsweise ist eine Wellenlänge entsprechend der Absorption zur Messung einer Konzentration von Körpergewebe und/oder eine Wellenlänge entsprechend der Absorption zur Messung einer Konzentration von Blut vorgesehen.

8. Messsystem nach einem der vorhergehenden Ansprüche, **dadurch gekennzeichnet, dass** mehrere emittierte Lichtwellen und mehrere empfangene Messwellensignale in der Messvorrichtung simultan geführt sind.

9. Messsystem nach einem der vorhergehenden Ansprüche, **dadurch gekennzeichnet, dass** die wenigstens eine Messvorrichtung durch eine nicht-invasive Messvorrichtung gegeben ist.

10. Messsystem nach einem der vorhergehenden Ansprüche,
**dadurch gekennzeichnet, dass** in der Auswerteeinheit ein numerisches Verfahren implementiert ist, das mittels eines linearen Gleichungssystems ein Gewebewasservolumen, die Konzentration von Blut und/oder eine Gewebewasserkonzentration und Konzentrationen von oxygeniertem Hämoglobin, desoxygeniertem Hämoglobin und/oder einer Gesamthämoglobinkonzentration ermittelt.

11. Messsystem nach einem der vorhergehenden Ansprüche,
**dadurch gekennzeichnet, dass** eine Markervorrichtung zum Einbringen eines Farbstoffs in einen Blutfluss des Körpergewebes vorgesehen ist und das Messsystem einen Modus zur Ermittlung einer Farbstoffkonzentration im Blutfluss aufweist.

12. Verfahren zur Messung wenigstens eines Parameters in einem Körpergewebe mittels eines Messsystems nach einem der Ansprüche 1 bis 11, die Verfahrensschritte umfassend
- Anbringen wenigstens einer Messvorrichtung am Körper,
- Emittieren und Detektieren von mindestens vier Lichtwellen mit unterschiedlichen Wellenlängen, wobei wenigstens eine der Wellenlängen der Lichtwellen im Bereich der Absorption eines Konstituenten des Körpergewebes liegt,
- Übermitteln der aus dem Körpergewebe empfangenen Messwellensignale an eine Auswerteeinheit und Aufarbeiten derselben,
- Bestimmen einer Pulsatilität der Messwellensignale, basierend auf Änderungen von Konzentrationswerten von Konstituenten im Körpergewebe,
- Bestimmen eines Druckparameters in dem Körpergewebe mittels eines Transformationsalgorithmus unter Verwendung einer Vergleichsoperation der Veränderung bzw. des Verlaufs einer Amplitude der Pulsatilität mit einer Referenz-Druck-Volumenkurve.

13. Verfahren zur Messung wenigstens eines Parameters in einem Körpergewebe mittels eines Messsystems nach Anspruch 12, wobei aus den Messwellensignalen mehrerer Lichtwellen unterschiedlicher Wellenlänge mit einem numerischen Verfahren gleichzeitig die Hämodynamik und die Konzentration von Konstituenten des Gehirns oder eines Muskels ermittelt wird.

## Claims

1. Measurement system for determining at least one pressure parameter in a bodily tissue in a brain or a muscle comprising:
- at least one measuring device to be applied to the body,
- an optical unit for emitting and detecting at least four light waves with different wavelengths, whereby at least one of the wavelengths of the light waves lies in the range of the absorption of a constituent of the bodily tissue,
- at least one light guide between optical unit and the at least one measuring device for transmission of light waves with different wavelengths, and
- an evaluation unit for evaluating measuring wave signals which are received and transmitted by the measuring device,
- whereby light waves with different wavelengths emitted by the optical unit are able to be radiated into an optical measurement volume in the bodily tissue by means of the at least one measuring device and the measuring wave signals received from the optical measurement volume by the measuring device are transmittable to the evaluation unit,
whereby
- the evaluation unit is designed to process the received measuring wave signals based on the absorption of the emitted light waves
- in order to determine a pulsatility of a concentration parameter measured in the measurement volume, and
whereby
- the evaluation unit comprises a transformation algorithm designed to determine a pressure parameter in the body tissue from the pulsatility of the concentration parameter by a comparison operation of the change or course of an amplitude of the pulsatility with a reference pressure-volume curve.

2. Measurement system according to claim 1, **characterized in that** at least one of the concentration values of the constituents is a concentration of oxygenated hemoglobin, deoxygenated hemoglobin and/or a total hemoglobin concentration.

3. Measurement system according to claim 1 or 2, **characterized in that** the pressure parameter is a parameter of intracranial pressure in the brain.

4. Measurement system according to claim 1 or 2, **characterized in that** the pressure parameter is a compartmental pressure of the muscle.

5. Measurement system according to one of the preceding claims, **characterized in that** one of the wavelengths of the emitted light waves is in the range of water absorption and another of the wavelengths is selected in the range of absorption of one of the constituents in the bodily tissue.

6. Measurement system according to one of the preceding claims, **characterized in that** at least four light waves having different wavelengths are able to be emitted from the optical light unit for determining four constituents in the bodily tissue, whereby a product of absorption coefficient and concentration value of the constituent for the four constituents in the bodily tissue is within one order of magnitude.

7. Measurement system according to one of the preceding claims, **characterized in that** for different constituents in the bodily tissue, a wavelength corresponding to the absorption by one of the constituents is provided for measuring a concentration of this constituent, preferably a wavelength corresponding to the absorption is provided for measuring a concentration of bodily tissue and/or a wavelength corresponding to the absorption is provided for measuring a concentration of blood.

8. Measurement system according to one of the preceding claims, **characterized in that** a plurality of emitted light waves and a plurality of received measuring wave signals are simultaneously guided in the measuring device.

9. Measurement system according to one of the preceding claims, **characterized in that** the at least one measuring device is given by a noninvasive measuring device.

10. Measurement system according to one of the preceding claims, **characterized in that** a numerical method is implemented in the evaluation unit, which determines a tissue water volume, the concentration of blood and/or a tissue water concentration and concentrations of oxygenated hemoglobin, deoxygenated hemoglobin and/or a total hemoglobin concentration by means of a linear system of equations.

11. Measurement system according to one of the preceding claims, **characterized in that** a marker device for introducing a dye into a blood flow of the bodily tissue is provided, and the measuring system has a mode for detecting a dye concentration in the blood flow.

12. Method for measuring at least one parameter in a bodily tissue by means of a measuring system according to any one of claims 1 to 11, comprising the method steps of
- Attaching at least one measuring device to the body,
- Emitting and detecting at least four light waves having different wavelengths, wherein at least one of the wavelengths of the light waves is in the range of absorption of a constituent of the bodily tissue,
- Transmitting the measuring wave signals received from the bodily tissue to an evaluation unit and processing them,
- Determining a pulsatility of the measuring wave signals, based on changes in concentration values of constituents in the bodily tissue,
- -determining a pressure parameter in the bodily tissue by means of a transformation algorithm using a comparison operation of the variation or respectively course of an amplitude of the pulsatility with a reference pressure-volume curve.

13. Method for measuring at least one parameter in a bodily tissue by means of a measuring system according to claim 12, whereby the hemodynamics and the concentration of constituents of the brain or of a muscle are determined simultaneously from the measuring wave signals of a plurality of light waves of different wavelengths by means of a numerical method.

## Revendications

1. Système de mesure pour la détermination d'au moins un paramètre de pression dans un tissu corporel du cerveau ou d'un muscle comprenant :
- au moins un dispositif de mesure à appliquer au corps,
- une unité optique pour l'émission et la détection d'au moins quatre ondes lumineuses ayant des longueurs d'onde différentes, au moins une des longueurs d'onde des ondes lumineuses se trouvant dans le domaine d'absorption d'un constituant du tissu corporel,
- au moins un guide optique entre l'unité optique et au moins un dispositif de mesure pour la transmission des ondes lumineuses ayant différentes longueurs d'onde, et
- une unité d'évaluation pour l'évaluation des signaux d'ondes de mesure qui sont reçus et transmis par le dispositif de mesure,
- les ondes lumineuses ayant différentes longueurs d'onde émises par l'unité optique pouvant être rayonnées dans un volume de mesure optique du tissu corporel au moyen dudit au moins un dispositif de mesure, et les signaux d'onde de mesure reçus par le volume de mesure optique par le dispositif de mesure pouvant être transmis à l'unité d'évaluation,
dans lequel
- l'unité d'évaluation est conçue pour traiter les signaux de mesure reçus en fonction de l'absorption des ondes lumineuses émises
- afin de déterminer une pulsatilité d'un paramètre de concentration mesuré dans le volume de mesure, et
dans lequel
- l'unité d'évaluation comprend un algorithme de transformation conçu pour déterminer un paramètre de pression dans le tissu corporel à partir de la pulsatilité du paramètre de concentration par une opération de comparaison de la variation ou de l'évolution d'une amplitude de la pulsatilité vis-à-vis d'une courbe pression-volume de référence.

2. Système de mesure selon la revendication 1, **caractérisé en ce qu'**au moins une des valeurs de concentration des constituants est une concentration d'hémoglobine oxygénée, d'hémoglobine désoxygénée et/ou une concentration d'hémoglobine totale.

3. Système de mesure selon la revendication 1 ou 2, **caractérisé en ce que** le paramètre de pression est un paramètre de pression intracrânienne dans le cerveau.

4. Système de mesure selon la revendication 1 ou 2, **caractérisé en ce que** le paramètre de pression est une pression compartimentale du muscle.

5. Système de mesure selon l'une des revendications précédentes, **caractérisé en ce qu'**une des longueurs d'onde des ondes lumineuses se trouve dans la gamme d'absorption de l'eau et une autre des longueurs d'onde est sélectionnée dans la gamme d'absorption de l'un des constituants du tissu corporel.

6. Système de mesure selon l'une des revendications précédentes, **caractérisé en ce qu'**au moins quatre ondes lumineuses ayant différentes longueurs d'onde peuvent être émises par l'unité de lumière optique pour déterminer quatre constituants du tissu corporel, le produit du coefficient d'absorption et de la valeur de concentration du constituant pour les quatre constituants du tissu corporel se situant à l'intérieur d'un même ordre de grandeur.

7. Système de mesure selon l'une des revendications précédentes, **caractérisé en ce que** pour différents constituants du tissu corporel, une longueur d'onde correspondant à l'absorption par l'un des constituants est prévue pour mesurer une concentration de ce constituant, de préférence une longueur d'onde correspondant à l'absorption est prévue pour la mesure d'une concentration de tissu corporel et/ou une longueur d'onde correspondant à l'absorption est prévue pour la mesure d'une concentration de sang.

8. Système de mesure selon l'une des revendications précédentes, **caractérisé en ce qu'**une pluralité d'ondes lumineuses émises et une pluralité de signaux d'ondes de mesure reçues sont guidées simultanément dans le dispositif de mesure.

9. Système de mesure selon l'une des revendications précédentes, **caractérisé en ce qu'**au moins un dispositif de mesure est constitué par un dispositif de mesure non invasif.

10. Système de mesure selon l'une des revendications précédentes, **caractérisé en ce qu'**un procédé numérique est implémenté dans l'unité d'évaluation, qui détermine un volume d'eau de tissu, la concentration de sang et/ou une concentration d'eau tissulaire et des concentrations d'hémoglobine oxygénée, d'hémoglobine désoxygénée et/ou une concentration d'hémoglobine totale au moyen d'un système linéaire d'équations.

11. Système de mesure selon l'une des revendications précédentes, **caractérisé en ce qu'**un dispositif de marquage pour introduire un colorant dans un flux sanguin du tissu corporel est fourni, et que le système de mesure possède un mode pour détecter une concentration de colorant dans le flux sanguin.

12. Procédé de mesure d'au moins un paramètre dans un tissu corporel au moyen d'un système de mesure selon l'une des revendications 1 à 11, comprenant les étapes de procédé de
- Fixation d'au moins dispositif de mesure au corps,
- Emission et détection d'au moins quatre ondes lumineuses ayant différentes longueurs d'onde, au moins une des longueurs d'onde des ondes lumineuses étant dans la gamme d'absorption d'un constituant du tissu corporel,
- Transmission des signaux d'ondes de mesure reçus par le tissu corporel à une unité d'évaluation et traitement de ces derniers ;
- Détermination d'une pulsatilité des signaux d'ondes de mesure, basés sur des changements de valeurs de concentration des constituants du tissu corporel,
- Détermination d'un paramètre de pression dans le tissu corporel au moyen d'un algorithme de transformation utilisant une opération de comparaison de la variation ou respectivement de l'évolution d'une amplitude de la pulsatilité vis-à-vis d'une courbe pression-volume de référence.

13. Procédé de mesure d'au moins un paramètre d'un tissu corporel au moyen d'un système de mesure selon la revendication 12, où l'hémodynamique et la concentration des constituants du cerveau ou d'un muscle sont déterminées simultanément à partir des signaux d'ondes de mesure d'une pluralité d'ondes lumineuses de différentes longueurs d'onde au moyen d'un procédé numérique.
